## Europäisches Patentamt

## European Patent Office

## Office européen des brevets

(19)

(11) Veröffentlichungsnummer: **0 042 969**
**B1**

(12) # EUROPÄISCHE PATENTSCHRIFT

(45) Veröffentlichungstag der Patentschrift:
13.02.85

(21) Anmeldenummer: 81103866.0

(22) Anmeldetag: 20.05.81

(51) Int. Cl.⁴: **C 12 N 9/96, C 12 Q 1/42**

(54) Verfahren zur Stabilisierung der alkalischen Phosphatase.

(30) Priorität: 24.06.80 DE 3023485

(43) Veröffentlichungstag der Anmeldung:
06.01.82 Patentblatt 82/1

(45) Bekanntmachung des Hinweises auf die Patenterteilung:
13.02.85 Patentblatt 85/7

(84) Benannte Vertragsstaaten:
BE CH DE FR GB IT LI NL SE

(56) Entgegenhaltungen:
CHEMICAL ABSTRACTS, Band 85, Nr. 15, 11. Oktober 1976, Seite 228, Nr. 105881w Columbus, Ohio, U.S.A. NIELS AGERGAARD: "In vitro inhibition and reactivation of the zinc metalloenzyme alkaline phosphatase in pigs"
CLINICAL CHEMISTRY, Band 26, Nr. 3, März 1980, Seiten 423-428 Easton, Pennsylvania, U.S.A. R. REJ et al.: "Effects of metal ions on the measurement of alkaline phosphatase activity"

(73) Patentinhaber: Merck Patent Gesellschaft mit beschränkter Haftung, Frankfurter Strasse 250, D-6100 Darmstadt (DE)

(72) Erfinder: Vormbrock, Rolf, Dr., Grundstrasse 9, D-6100 Darmstadt (DE)
Erfinder: Helger, Roland, Dr., Ludwigshöhstrasse 85, D-6100 Darmstadt (DE)

## Beschreibung

Die vorliegende Erfindung betrifft Verfahren zur Stabilisierung der alkalischen Phosphatase in Pufferlösungen, wie sie in der klinischen Chemie zur Bestimmung der Enzymaktivität der alkalischen Phosphatase und ihrer Isoenzyme verwendet werden.

Die Bestimmung der enzymatischen Aktivität der alkalischen Phosphatase (EC 3.1.3.1) im Serum oder Plasma spielt eine wichtige Rolle in der klinischen Diagnostik. Diese Bestimmung wird in gepufferter wäßriger Lösung durchgeführt, wobei der pH-Wert der Lösung über 8 liegen sollte. Als Puffer wurden zunächst Barbitonpuffer (pH 8,6), später Carbonat-Hydrogencarbonat-Puffer (pH 9,9) oder Glycin-Natronlauge-Puffer (pH 10,5) verwendet. Nachdem gefunden worden war, daß Aminoalkohole, die als Phosphorylakzeptoren fungieren können, in hoher Konzentration die enzymatische Aktivität der alkalischen Phosphatase erhöhen, wurde vor allem Diethanolamin und 2-Amino-2-methylpropanol-(1) als Puffersubstanzen für die Bestimmung der enzymatischen Aktivität der alkalischen Phosphatase in der klinischen Chemie eingesetzt. Diese Puffersubstanzen haben jedoch den Nachteil, daß sie Verunreinigungen enthalten können, die die alkalische Phosphatase inhibieren oder inaktivieren. Im Falle des Diethanolamins handelt es sich um Ethanolamin, das durch aufwendige Rektifikation abgetrennt werden kann. Im Falle des 2-Amino-2-methylpropanol-(1) ist aus einer Reihe von Verunreinigungen ein substituiertes Ethylenderivat von zur Zeit noch nicht vollständig aufgeklärter Struktur als Inaktivator der alkalischen Phosphatase identifiziert worden [Clin. Chem. 24, 1611 (1978)].

Aus Clin. Chim. Acta 98, 61 (1979) ist bekannt, daß die Inaktivierung der alkalischen Phosphatase im 2-Amino-2-methylpropanol-(1)-Puffer vermieden werden kann, wenn dem Puffer Zinksalze zugesetzt werden. Dieses Verfahren hat jedoch den Nachteil, daß bei überoptimalen Konzentrationen an Zinkionen die alkalische Phosphatase dennoch inhibiert wird, so daß nur bei genauer Dosierung der Zinksalze eine maximale Enzymaktivität der alkalischen Phosphatase gewährleistet ist. Die optimale Konzentration an zuzusetzenden Zinkionen ist jedoch für jede Charge des Puffers verschieden.

Der Erfindung liegt die Aufgabe zugrunde, ein Verfahren zur Stabilisierung der alkalischen Phosphatase in Pufferlösungen zur Verfügung zu stellen, durch das eine Inhibierung der enzymatischen Aktivität verhindert wird und das stets optimale und reproduzierbare Ergebnisse liefert.

Überraschenderweise wurde gefunden, daß komplex gebundene Zinkionen ebensogut wie Zinksalze die enzymatische Aktivität der alkalischen Phosphatase im 2-Amino-2-methylpropanol-(1)-Puffer stabilisieren können. Dies ist vor allem deshalb so überraschend, als gerade Komplexbildner für die Inhibierung verantwortlich sind und andererseits bekannt ist, daß die Inhibierung von alkalischer Phosphatase durch Ethylendiamintetraacetat nur teilweise und nur durch Zugabe ganz bestimmter Konzentrationen von Zinkionen wieder aufgehoben werden kann. Die Vorteile der neuen Stabilisatoren liegen darin, daß im Gegensatz zum Zinksulfat und anderen Zinksalzen bei Stabilisatorkonzentrationen von mehr als 0,1 mmol/l keine Inhibierung der Enzymaktivität der alkalischen Phosphatase mehr auftritt. Der Bereich optimaler Stabilisatorkonzentration ist sehr breit, so daß eine einzige Stabilisatorkonzentration für verschiedene Chargen von Puffer unterschiedlicher Qualität verwendbar ist. Bei der Verwendung von Zinkkomplexen zur Stabilisierung der alkalischen Phosphatase entfällt somit die Optimierung der Stabilisatorkonzentration für die jeweils vorliegende Puffercharge.

Gegenstand der Erfindung ist ein Verfahren zur Stabilisierung der alkalischen Phosphatase in Pufferlösungen, das dadurch gekennzeichnet ist, daß der Pufferlösung ein wasserlöslicher Zinkkomplex zugesetzt wird, vorzugsweise ein Verfahren zur Stabilisierung der alkalischen Phosphatase in 2-Amino-2-methylpropanol-(1)-Puffer, das dadurch gekennzeichnet ist, daß dem Puffer ein Komplex aus Zinkionen und einem Chelatbildner zugesetzt wird.

Die Bestimmung der alkalischen Phosphatase mit Hilfe des neuen Stabilisators erfolgt auf übliche Weise: Einer thermostatisierten Pufferlösung, die den Stabilisator und Magnesiumionen enthält, wird die zu untersuchende Probe zugegeben. Nach einer bestimmten Inkubationszeit wird das Substrat zugesetzt und in einem Photometer die Extinktionsänderung pro Zeiteinheit registriert, die der Enzymaktivität proportional ist.

Der Stabilisator besteht aus einem Zinkchelatkomplex. Dieser Komplex kann entweder als solcher der Puffersubstanz oder der Pufferlösung zugesetzt werden oder man kann dem Puffer beide Bestandteile des Komplexes gleichzeitig oder nacheinander zugeben.

Geeignete Zinkverbindungen zur Herstellung des Komplexes sind insbesondere alle Zinksalze, die in Wasser oder in einer Lösung des Komplexbildners löslich sind und die die enzymatische Bestimmung der alkalischen Phosphatase nicht stören, wie Zinksulfat, Zinkacetat, Zinknitrat oder auch Zinkhydroxid usw., vorzugsweise Zinksulfat.

Geeignete Chelatbildner zur Bildung eines wasserlöslichen Zinkkomplexes sind z. B. Ethylendiamintetraacetat, trans-1,2-Diaminohexantetraacetat, Diethylentriaminpentaacetat, Ethylenglycol-bis-($\beta$-aminoethylether)-N,N'-tetraacetat usw., vorzugsweise Ethylendiamintetraacetat. Die Chelatbildner können dabei entweder in Form ihrer Salze oder auch in der Säureform eingesetzt werden.

Die Herstellung des Zinkkomplexes erfolgt durch Mischung des Chelatbildners mit der Zinkverbindung im molaren Verhältnis von etwa 1 : 1. Dabei ist es nicht kritisch, ob man den Zinkkomplex als Substanz, z. B. ZnEDTA · 4 H$_2$O, dem Puffer zusetzt oder äquimolare Mengen an z. B. Zinksulfat und Ethylendiamintetraacetat nacheinander in den Puffer gibt, so daß sich der Komplex in der Pufferlö-

2

sung bilden kann.

Das molare Verhältnis von Komplex zu Puffer kann im Bereich von $1 : 10^6$ bis $1 : 10$ variiert werden; bevorzugt ist ein Bereich von etwa $1 : 10^4$ bis $1 : 10^3$.

Die Erfindung wird durch die folgenden Beispiele näher erläutert.

### Beispiel 1

Untersuchung des Einflusses verschiedener Stabilisatorkonzentrationen auf die enzymatische Aktivität der alkalischen Phosphatase. Vergleich der stabilisierenden Wirkung von Zink-Ethylendiamintetraacetat (ZnEDTA) mit Zinksulfat:

In eine Küvette mit 1 cm Schichtdicke wurden 2,8 ml Pufferlösung der folgenden Zusammensetzung pipettiert:

| | |
|---|---|
| 2-Amino-2-methylpropanol-(1) | 0,96 mol/l, |
| Magnesiumsulfat | 1,0 mmol/l, |
| Stabilisator ($ZnSO_4$ bzw. ZnEDTA) | $10^{-5}$ bis $10^{-2}$ mol/l. |

Die Lösung wurde auf eine Temperatur von 30° C gebracht und anschließend wurden 0,1 ml Serum zugegeben. Die Lösung wurde gemischt und bei 30° C vorinkubiert. Die Dauer der Vorinkubation betrug bei Meßreihe a) 90 Sekunden, bei Meßreihe b) 10 Minuten. Die Inkubation wurde durch Zugabe von 0,1 ml einer Lösung des Dinatriumsalzes von p-Nitrophenylphosphat in einer Konzentration von 0,48 mol/l beendet. Die Lösung wurde gemischt und aus der zeitlichen Änderung der Extinktion bei 405 nm wurde die Aktivität der alkalischen Phosphatase berechnet.

| Stabilisator- konzentration [mol/l] | Enzymaktivität [U/l] a) Vorinkubation 90 sec | | b) Vorinkubation 10 min | |
|---|---|---|---|---|
| | $ZnSO_4$ | ZnEDTA | $ZnSO_4$ | ZnEDTA |
| $3 \cdot 10^{-5}$ | 201 | 184 | 171 | 162 |
| $3 \cdot 10^{-5}$ | 209 | 191 | 186 | 184 |
| $3 \cdot 10^{-4}$ | 206 | 202 | 200 | 200 |
| $3 \cdot 10^{-4}$ | 201 | 205 | 205 | 202 |
| $3 \cdot 10^{-3}$ | 157 | 204 | 151 | 203 |
| $3 \cdot 10^{-3}$ | 11 | 200 | 18 | 199 |
| $3 \cdot 10^{-2}$ | 0 | 195 | 0 | 196 |

Aus den Tabellen geht eindeutig hervor, daß für das Zinksalz im Vergleich zum ZnEDTA nur ein enger Konzentrationsbereich optimaler Stabilisatorwirkung existiert. Etwa ab eine Stabilisatorkonzentration von $10^{-3}$ mol/l wirkt Zinksulfat als Inhibitor und bei einer Konzentration von $10^{-2}$ mol/l Zinksulfat ist die Enzymaktivität auf 0 abgesunken, während im Falle von ZnEDTA noch bei einer optimalen Enzymaktivität gemessen werden kann.

### Beispiel 2

Untersuchung der Wirkung der Stabilisatoren $ZnSO_4$ und ZnEDTA in Puffern unterschiedlicher Inhibitorkonzentration:

Bei der fraktionierten Destillation von 2-Amino-2-methylpropanol-(1) wurden Fraktionen erhalten, die eine mehr oder weniger starke Inaktivierung der alkalischen Phosphatase bewirkten. Diese Fraktionen wurden für die Bestimmung der enzymatischen Aktivität der alkalischen Phosphatase in Gegenwart von $ZnSO_4$ bzw. ZnEDTA verwendet. Die Bestimmung wurde analog Beispiel 1 mit einer Inkubationszeit von 90 Sekunden durchgeführt.

In der nachstehenden Tabelle ist die Wirkung der Stabilisatoren bei einer Konzentration von $10^{-3}$ mol/l einander gegenübergestellt. Die enzymatische Aktivität der alkalischen Phosphatase ist in U/l angegeben.

0 042 969

| Puffer | Enzymaktivität [U/l] | |
| --- | --- | --- |
| | ZnSO$_4$ | ZnEDTA |
| Fraktion 1 | 41 | 195 |
| Fraktion 2 | 43 | 202 |
| Rückstand | 203 | 204 |

Die Tabelle zeigt, daß lediglich im Destillationsrückstand bei extrem erhöhter Konzentration an Verunreinigung beide Stabilisatoren bei einer Konzentration von 10$^{-3}$ mol/l gleichwertig sind. Die gleiche Stabilisatorkonzentration führt jedoch bei den weniger verunreinigten Fraktionen des Puffers im Falle des Zinksulfats zu einer Inhibierung der enzymatischen Aktivität der alklaischen Phosphatase von etwa 80% gegenüber ZnEDTA.

Beispiel 3

Untersuchung der Eignung verschiedener Zinkkomplexe zur Stabilisierung der alkalischen Phosphatase in 2-Amino-2-methylpropanol-(1)-Puffer:

Die Bestimmung wurde analog Beispiel 1 durchgeführt, wobei die Stabilisatorkonzentration in allen Fällen 10$_{,}$$^4$ mol/l berug; die Vorinkubationszeit betrug 90 Sekunden bzw. 10 Minuten. Die Ergebnisse sind in der nachstehenden Tabelle dargestellt:

| Zinkkomplexligand | Enzymaktivität [U/l] | |
| --- | --- | --- |
| | Vorinkubation a) 90 sec | Vorinkubation b) 10 min |
| Ethylendiamintetraacetat | 212 | 214 |
| trans-1,2-Diaminohexantetraacetat | 213 | 206 |
| Diethylentriaminpentaacetat | 216 | 218 |
| Ethylenglycol-bis-($\beta$-amino-ethylether)-N,N'-tetraace-tat | 207 | 217 |
| ohne Stabilisator | 180 | 82 |

Es zeigt sich, daß alle untersuchten Zinkkomplexe zur Stabilisierung der alkalischen Phosphatase geeignet sind.

**Patentansprüche**

1. Verfahren zur Stabilisierung der alkalischen Phosphatase in Pufferlösungen, dadurch gekennzeichnet, daß der Pufferlösung ein wasserlöslicher Zinkkomplex zugesetzt wird.

2. Verfahren zur Stabilisierung der alkalischen Phosphatase in 2-Amino-2-methylpropanol-(1)-Puffer, dadurch gekennzeichnet, daß dem Puffer ein Komplex aus Zinkionen und einem Chelatbildner zugesetzt wird.

3. Verfahren nach den Ansprüchen 1 und 2, dadurch gekennzeichnet, daß dem Puffer Zink-Ethylendiamintetraacetat zugesetzt wird

4. Verfahren nach den Ansprüchen 1 bis 3, dadurch gekennzeichnet, daß dem Puffer eine Zinkverbindung und ein Chelatbildner zugesetzt werden.

**Claims**

1. Process for the stabilisation of alkaline phosphatase in buffer solutions, characterised in that a water-soluble zinc complex is added to the buffer solution.

2. Process for the stabilisation of alkaline phosphatase in 2-amino-2-methylpropan-1-ol buffer,

**0 042 969**

characterised in that a complex of zinc ions and a chelating agent is added to the buffer.

3. Process according to Claims 1 and 2, characterised in that zinc ethylenediaminetetraacetate is added to the buffer.

4. Process according to Claims 1 to 3, characterised in that a zinc compound and a chelating agent are added to the buffer.

**Revendications**

1. Procédé pour la stabilisation de la phosphatase alcaline dans des solutions tampons, caractérisé en ce qu'on ajoute un complexe de zinc hydrosoluble à la solution tampon.

2. Procédé pour la stabilisation de la phosphatase alcaline dans un tampon de 2-amino-2-méthylpropanol-(1), caractérisé en ce qu'on ajoute, au tampon, un complexe d'ions zinc et d'un agent formateur de chélate.

3. Procédé suivant les revendications 1 et 2, caractérisé en ce qu'on ajoute, au tampon, un complexe de zinc/tétracétate d'éthylène-diamine.

4. Procédé suivant les revendications 1 à 3, caractérisé en ce qu'on ajoute, au tampon, un composé de zinc et un agent formateur de chélate.